# EUROPÄISCHE PATENTSCHRIFT

(11) **EP 0 988 306 B1**
(45) Veröffentlichungstag und Bekanntmachung des Hinweises auf die Patenterteilung: **09.07.2003**
(21) Anmeldenummer: 98929405.3
(22) Anmeldetag: 29.05.1998
(51) Int. Cl.: C07D 495/04, A61K 31/495

(54) **3-SUBSTITUIERTE 3,4-DIHYDRO-THIENO 2,3-D]PYRIMIDIN-DERIVATE, IHRE HERSTELLUNG UND VERWENDUNG**
3-SUBSTITUTED 3,4 DIHYDRO-THIENO 2, 3-D]PYRIMIDINE DERIVATIVES AND PRODUCTION AND USE OF THE SAME
DERIVES DE 3,4-DIHYDRO-THIENO 2,3-D]PYRIMIDINE 3-SUBSTITUES, LEUR PREPARATION ET LEUR UTILISATION

(30) Priorität: 13.06.1997 DE 19724980
(43) Veröffentlichungstag der Anmeldung: 29.03.2000
(73) Patentinhaber: Abbott GmbH & Co. KG, 65205 Wiesbaden (DE)
(72) Erfinder: STEINER, Gerd, D-67281 Kirchheim (DE); DULLWEBER, Uta, D-67227 Frankenthal (DE); STARCK, Dorothea, D-67059 Ludwigshafen (DE); BACH, Alfred, D-69123 Heidelberg (DE); WICKE, Karsten, D-67112 Altrip (DE); TESCHENDORF, Hans-Jürgen, D-67373 Dudenhofen (DE); GARCIA-LADONA, Francisco-Javier, D-76870 Kandel (DE); EMLING, Franz, D-67065 Ludwigshafen (DE)
(74) Vertreter: Grünecker, Kinkeldey, Stockmair & Schwanhäusser Anwaltssozietät
(86) Internationale Anmeldenummer: EP9803230
(87) Internationale Veröffentlichungsnummer: WO98056792

(56) Entgegenhaltungen:
- EP-A- 0 329 168
- WO-A-98/11110
- US-A- 4 835 157

## Beschreibung

Die Erfindung betrifft neue 3,4-Dihydro-thieno[2,3-d]pyrimidin-Derivate, deren Herstellung und Verwendung zur Herstellung von Pharmawirkstoffen.

Die klassischen Antidepressiva und auch die neueren selektiven Serotonin Reuptakehemmer (SSRIs) entfalten ihre antidepressive Wirkung unter anderem durch die Inhibierung der aktiven Wiederaufnahme des Transmitters in die präsynaptischen Nervenendigungen. Leider tritt dabei die antidepressive Wirkung erst nach einer Behandlung von mindestens 3 Wochen ein, zudem sind ca 30 % der Patienten therapieresistent.

Die Blockade von präsynaptischen Serotonin-Autorezeptoren erhöht durch Aufhebung der negativen Kopplung die Serotoninfreisetzung und damit die aktuelle Transmitterkonzentration im synaptischen Spalt. Dieser Anstieg der Transmitterkonzentration gilt als das antidepressive Wirkprinzip. Dieser Wirkmechanismus unterscheidet sich von den bisher bekannten Antidepressiva, die zugleich die präsynaptischen und somatodendritischen Autorezeptoren aktivieren und deshalb erst nach Desensibilisierung dieser Autorezeptoren zum verzögerten Wirkungseinstritt führen. Die direkte Autorezeptor-Blockade umgeht diesen Effekt.

Nach bisherigem Wissen handelt es sich bei dem präsynaptischen Serotonin-Autorezeptor um den 5-HT_{1B}-Subtyp (Fink et al., Arch. Pharmacol. 352 (1995), S. 451). Dessen selektive Blockade durch 5-HT_{1B/D}-Antagonisten erhöht die Serotonin-Freisetzung im Gehirn: G.W. Price et al., Behavioural Brain Research 73 (1996), S. 79-82; P.H. Hutson et al., Neuropharmacology Vol. 34, No. 4 (1995), S. 383-392.

Der selektive 5-HT_{1B}-Antagonist GR 127 935 vermindert jedoch überraschenderweise die Serotonin-Freisetzung im Cortex nach systemischer Gabe. Eine Erklärung könnte die Stimulierung von somatodendritischen 5-HT_{1A}-Rezeptoren in der Raphe Region durch das freigesetzte Serotonin sein, die die Feuerrate serotonerger Neuronen und damit die Serotonin-Ausschüttung hemmt (M. Skingle et al., Neuropharmacology Vol. 34 No. 4 (1995), S. 377-382, S. 393-402).

Eine Strategie zur Umgehung der autoinhibitorischen Effekte in serotonergen Ursprungsgebieten verfolgt also die Blockade der präsynaptischen 5-HT_{1B} Rezeptoren. Diese Hypothese wird gestützt durch die Beobachtung, daß der Einfluß von Paroxetine auf die Serotonin-Freisetzung im dorsalen Raphe Nucleus der Ratte durch den 5-HT_{1B}-Rezeptor Antagonisten GR 127 935 potenziert wird (Davidson and Stamford, Neuroscience Letts., 188 (1995),41).

Die zweite Strategie schließt die Blockade beider Typen von Autorezeptoren mit ein, nämlich die 5-HT_{1A}-Rezeptoren, um das neuronale Feuern zu verstärken, und die 5-HT_{1B}-Rezeptoren, um die terminale Serotonin Freisetzung anzuheben (Starkey and Skingle, Neuropharmacology 33 (3-4) (1994),393).

5-HT_{1B/D}-Antagonisten allein oder gekoppelt mit einer 5-HT_{1A}-Rezeptor antagonistischen Komponente sollten deshalb vermehrt die Serotonin-Freisetzung im Gehirn erhöhen und könnten deshalb Vorteile in der Therapie von Depressionen und verwandten psychischen Krankheiten beinhalten.

Es wurde nun gefunden, daß 3-substituierte 3,4-Dihydrothieno-[2,3-d]pyrimidin-Derivate der Formel I 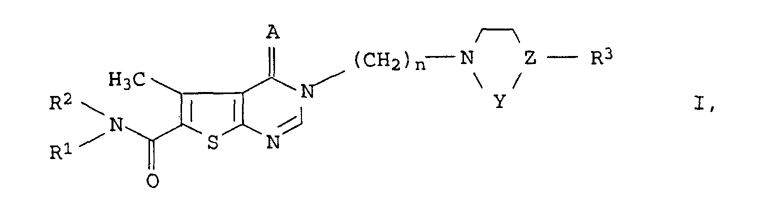 worin
- R¹ und R²: ein Wasserstoffatom oder eine C₁-C₄-Alkylgruppe bedeutet,
- R³: eine gegebenenfalls durch Halogenatome, C₁-C₄-Alkyl, Trifluormethyl-, Trifluormethoxy-, Hydroxy-, C₁-C₄-Alkoxy-, Amino-, Monomethylamino-, Dimethylamino-, Cyano- oder Nitrogruppen mono- oder disubstituierte Phenyl-, Pyridyl-, Pyrimidinyl- oder Pyrazinyl-Gruppe darstellt, die gegebenenfalls mit einem Benzolkern, der gegebenenfalls durch Halogen-atome, C₁-C₄-Alkyl-, Hydroxy-, Trifluormethyl-, C₁-C₄-Alkoxy-, Amino-, Cyano- oder Nitrogruppen mono- oder disubstituiert sein kann und gegebenenfalls 1 Stickstoffatom enthalten kann, oder mit einem 5- oder 6-gliedrigen Ring, der 1-2 Sauerstoffatome enthalten kann, anelliert sein kann,
- A: NH oder ein Sauerstoffatom darstellt,
- Y: CH₂, CH₂-CH₂, CH₂-CH₂-CH₂ oder CH₂-CH
ist,
- Z: ein Stickstoffatom, Kohlenstoffatom oder CH darstellt, wobei die Bindung zwischen Y und Z auch eine Doppelbindung sein kann,
und n die Zahl 2,3 oder 4 bedeutet
und deren Salze mit physiologisch verträglichen Säuren wertvolle pharmakologischen Eigenschaften besitzen.

Bevorzugt sind insbesonders Verbindungen, in denen
- R¹ und R²: Methyl
- R²: o-Methoxyphenyl, 1-Naphthyl, 2-Methoxy-1-naphthyl, 2-Methyl-1-naphthyl
- A: ein Sauerstoffatom
- Y: CH₂-CH₂
- Z: ein Stickstoffatom
und n die Zahl 2 und 3 bedeuten.

Die erfindungsgemäßen Verbindungen der Formel I lassen sich herstellen, indem man eine Verbindung der Formel II 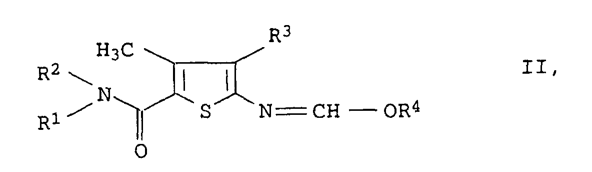 in der R₁ die oben angegebene Bedeutung hat, R³ eine Cyanogruppe oder eine C₁₋₃-Alkyl-carbonsäureestergruppierung darstellt und R⁴ C₁₋₃-Alkyl bedeutet, mit einem primären Amin der Formel III 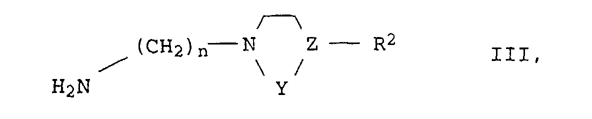 worin R³ die oben angegebene Bedeutung hat, umsetzt und die so erhaltene Verbindung gegebenenfalls in das Säureadditionssalz einer physiologisch verträglichen Säure überführt.

Die Umsetzung erfolgt zweckmäßig in einem inerten organischen Lösungsmittel, insbesondere einem niederen Alkohol, z.B. Methanol oder Ethanol, oder einem cyclischen gesättigten Ether, insbesondere Tetrahydrofuran oder Dioxan.

Die Umsetzung erfolgt in der Regel bei Temperaturen von 20 bis 110°C, insbesondere von 60 bis 90°C, und ist im allgemeinen innerhalb von 1 bis 10 Stunden beendet.

Oder man setzt eine Verbindung der Formel II 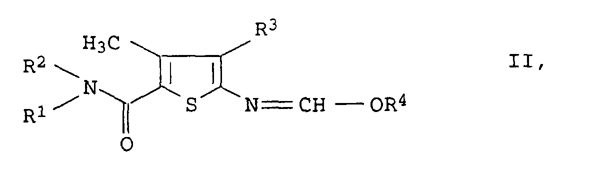 in der R₁ die oben angegebene Bedeutung hat, R³ eine Cyanogruppe oder eine C₁₋₃-Alkyl-carbonsäureestergruppierung darstellt und R⁴ C₁₋₃-Alkyl bedeutet, mit einem primären Aminoalkohol der Formel IV 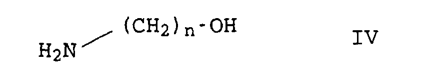 in einem inerten Lösungsmittel, vorzugsweise Alkoholen wie z.B. Ethanol, bei Temperaturen zwischen 60° und 120°C zum Cyclisierungsprodukt V (X=OH) um 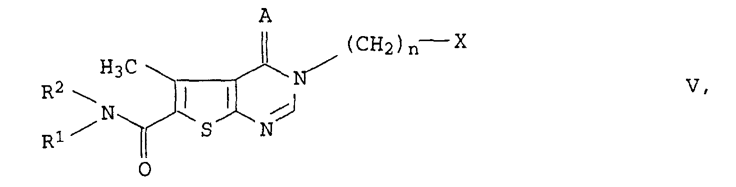 das anschließend mit einem Halogenierungsmittel, wie z.B. Thionylchlorid oder Bromwasserstoffsäure, in einem organischen Lösungsmittel wie einem Halogenkohlenwasserstoff oder ohne Lösungsmittel bei Temperaturen zwischen Raumtemperatur und 100°C in das entsprechende Halogenderivat V (X=C1, Br) überführt wird. Zuletzt setzt man das Halogenderivat der Formel V (X=C1, Br) mit einem Amin der allgemeinen Formel VI 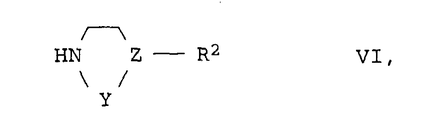 worin Y, Z und R² die oben angegebenen Bedeutungen haben, zum erfindungsgemäßen Endprodukt der Formel I um. Diese Umsetzung verläuft am besten in einem inerten organischen Lösungsmittel, vorzugsweise Toluol oder Xylol, in Gegenwart einer Base, wie z.B. Kaliumcarbonat oder Kaliumhydroxyd, bei Temperaturen zwischen 60°C und 150°C.

Die erfindungsgemäßen Verbindungen der Formel I können entweder durch Umkristallisation aus den üblichen organischen Lösungsmitteln, bevorzugt aus einem niederen Alkohol, wie Ethanol, umkristallisiert oder durch Säulenchromatographie gereinigt werden.

Die freien 3-substituierten Pyrido [3',4':4,5] thieno [2,3-d]pyrimidin-Derivate der Formel I können in üblicher Weise in die Säureadditionssalze einer Lösung mit der stöchiometrischen Menge der entsprechenden Säure. Pharmazeutisch verträgliche Säuren sind beispielsweise Salzsäure, Phosphorsäure, Schwefelsäure, Methansulfonsäure, Amidosulfonsäure, Maleinsäure, Fumarsäure, Oxalsäure, Weinsäure oder Zitronensäure.

Die Erfindung betrifft dementsprechend auch ein therapeutisches Mittel, gekennzeichnet durch einen Gehalt an einer Verbindung der Formel I oder deren pharmakologisch verträgliches Säureadditionssalz als Wirkstoff neben üblichen Träger- und Verdünnungsmitteln, sowie die Verwendung der neuen Verbindungen bei der Bekämpfung von Krankheiten.

Die erfindungsgemäßen Verbindungen können in üblicher Weise oral oder parenteral, intravenös oder intramuskulär verabfolgt werden.

Die Dosierung hängt vom Alter, Zustand und Gewicht des Patienten sowie von der Applikationsart ab. In der Regel beträgt die tägliche Wirkstoffdosis zwischen etwa 1 und 100 mg/kg Körpergewicht bei oraler Gabe und zwischen 0,1 und 10 mg/kg Körpergewicht bei parenteraler Gabe.

Die neuen Verbindungen können in gebräuchlichen galenischen Applikationsformen fest oder flüssig angewendet werden, z.B. als Tabletten, Filmtabletten, Kapseln, Pulver, Granulate, Dragees, Suppositorien, Lösungen, Salben, Cremes oder Sprays. Diese werden in üblicher Weise hergestellt. Die Wirkstoffe können dabei mit den üblichen galenischen Hilfsmitteln wie Tablettenbindern, Füllstoffen, Konservierungsmitteln, Tablettensprengmitteln, Fließreguliermitteln, Weichmachern, Netzmitteln, Dispergiermitteln, Emulgatoren, Lösungsmitteln, Retardierungsmitteln, Antioxidantien und/oder Treibgasen verarbeitet werden (vgl. H. Sucker et. al: Pharmazeutische Technologie, Thieme-Verlag, Stuttgart, 1978). Die so erhaltenen Applikationsformen enthalten den Wirkstoff normalerweise in einer Menge von 1 bis 99 Gew.-%.

Die als Ausgangsstoffe für die Synthese der neuen Verbindungen benötigten Substanzen der Formel II bis VI sind bekannt oder lassen sich gemäß den in der Literatur beschriebenen Herstellmethoden aus analogen Ausgangsmaterialien synthetisieren (F. Sauter und P. Stanetty, Monatsh. Chem. (1975), 106(5), 1111-1116; K. Gewald et al, Chem. Ber. 99, 94-100 (1966), Patentanmeldung DE 196 36769.7).

Die erfindungsgemäßen Verbindungen weisen eine hohe Affinität zu den Serotoninrezeptoren 5-HT_{1B}, 5-HT_{1D} und 5-HT_{1A} auf. Die Affinität zu diesen Rezeptoren ist dabei etwa gleich groß, zumindest in der gleichen Größenordnung. Darüberhinaus weisen einige der erfindungsgemäßen Verbindungen eine gute Serotonin-Reuptake Hemmung auf- ein Prinzip, das bei den meisten Antidepressiva verwirklicht ist.

Diese Verbindungen eignen sich als Arzneimittel zur Behandlung von Krankheitszuständen, bei denen die Serotoninkonzentration erniedrigt ist, und bei denen man im Rahmen einer Therapie gezielt die Aktivität der präsynaptischen Rezeptoren 5-HT_{1B}, 5-HT_{1A}, 5-HT_{1D} blockieren möchte, ohne dabei andere Rezeptoren stark zu beeinflussen. Solch ein Krankheitszustand ist beispielsweise die Depression.

Die Verbindungen der vorliegenden Erfindung können auch für die Behandlung von zentralnervös bedingten Gemütsstörungen wie saisonale affektive Störungen und Dysthymie von Nutzen sein. Dazu gehören auch Angstzustände wie generalisierte Angst, Panikanfälle, Soziophobie, Zwangsneurosen und post-traumatische Stress-Symptome, Gedächtnisstörungen einschließlich Demenz, Amnesien und altersbedingter Gedächtnisschwund sowie psychogene Eßstörungen wie Anorexia nervosa und Bulimia nervosa.

Die erfindungsgemäßen Verbindungen können außerdem für die Behandlung endokriner Erkrankungen wie Hyperprolaktinämie sowie für die Behandlung von Gefäßspasmen (insbesondere der Hirngefäße), Hypertonie und gastrointestinalen Störungen, die mit Motilitätsund Sekretionsstörungen einhergehen, von Nutzen sein. Ein weiteres Anwendungsgebiet sind Sexualstörungen.

Die folgenden Beispiele dienen zur Erläuterung der Erfindung:

### A Herstellung der Ausgangsmaterialien

a) 2-Amino-3-carboethoxy-5-methyl-5-dimethylcarbamoyl-thiophen
   Zu 100 g (775 mM) Acetessigsäuredimethylamid in 400 ml Ethanol wurden 82,8 ml (775 mM) Cyanessigsäureethylester und 24,8 g (755 mM) Schwefelpulver zugegeben und anschließend unter gutem Rühren und unter Stickstoffatmospähre 90 ml (647 mM) Triethylamin zugetropft. Nach 1 h erhitzte man den Ansatz 8 h auf Rückfluß und ließ noch über Nacht bei Raumtemperatur nachrühren. Man engte den Ansatz im Vakuum ein, nahm den Rückstand mit 2 1 Wasser auf, stellte auf pH=9 und extrahierte zweimal mit Methylenchlorid. Nach Trocknen und Einengen der organischen Phase reinigte man das Rohprodukt (70 g) durch Lösen in 200 ml siedendem Essigester. Die über Nacht unter Rühren ausgefallenen Festkörper saugte man nach Kühlen im Eisbad ab und wusch mehrmals mit kaltem Essigester nach. Man isolierte 39,0 g (20 %) Produkt als graue Festkörper mit Schmp. 122-124°C.
b) 2-Ethoxymethylen-amino-3-carboethoxy-4-methyl-5-dimethylcarbamoyl-thiophen
   30,6 g (119 mM) 2-Amino-3-carboethoxy-4-methyl-5-dimethylcarbamoyl-thiophen in 150 ml Triethylorthoformiat wurden mit 2,0 ml Acetanhydrid versetzt und unter Stickstoff 2 h am Rückfluß gekocht. Man engte danach den Ansatz bei 80°C am Rotationsverdampfer ganz ein. Man isolierte 35,6 g (96 %) Rohprodukt als dunkles Öl, das für die weitere Umsetzung genügend rein ist.
c) 3-(2-Hydroxy-ethyl)-5-methyl-6-dimethylcarbamoylthieno[2,3-d]pyrimidin-4-on
   35,6 g (114 mM) 2-Ethoxymethylen-amino-3-carboethoxy-5-methyl-5-dimethylcarbamoyl-thiophen in 200 ml Ethanol wurden mit 8,0 ml (133 mM) Ethanolamin versetzt und 2 h am Rückfluß gekocht. Anschließend engte man den Ansatz im Vakuum ein. Man isolierte 29.9 g (93 %) dunkles viskoses Öl.
d) 3-(2-Chlor-ethyl)-5-methyl-6-dimethylcarbamoylthieno[2,3-d]pyrimidin-4-on
   29,9 g (106 mM) 3-(2-Hydroxy-ethyl)-5-methyl-6-dimethylcarbamoyl-thieno[2,3-d]pyrimidin-4-on in 200 ml 1,2-Dichlorethan wurden auf Rückfluß erhitzt (langsame Auflösung) und anschließend 12,7 ml (175 mM) Thionylchlorid in 20 ml 1,2-Dichlorethan zugetropft. Nach 1 h Rückflußkochen engte man das Reaktionsgemisch nach dem Abkühlen ein. Das Rohprodukt verteilte man zwischen Methylenchlorid und Wasser bei pH=9. Nach Trocknen und Einengen der organischen Phase isolierte man 44,1 g (83 %) Produkt als dunkles Öl, das durch Säulenchromatographie (Kieselgel, Laufmittel Essigester) gereinigt wurde. Man isolierte 23,8 g (76 %) Produkt mit Schmp. 120-122°C.
   Analog der Vorschriften a) bis d) können andere C₁-C₄-Mono- bzw. Dialkyl-carbamoyl-Derivate der allgemeinen Formel II und V hergestellt werden.
e) N-(1-Naphthyl)-piperazin
   Zu einer Mischung aus 5,4 g (24,2 mM) Palladiumacetat und 14,7 g (48,3 mM) Tri-o-tolylphosphin in 500 ml Xylol wurden 83,2 g (966 mM) Piperazin, 38,0 g (339 mM) Kalium-tert.-butylat und 50,0 g (241 mM) 1-Bromnaphthalin zugegeben und die Reaktionsmischung 10 h unter gutem Rühren und unter Stickstoffatmosphäre auf Rückfluß erhitzt. Danach verdünnte man den Ansatz mit Methylenchlorid, filtrierte die unlöslichen Rückstände ab und engte das Filtrat ein. Das Rohprodukt reinigte man durch Säulenchromatographie (Kieselgel, Laufmittel THF/Methanol/Ammoniak 85/13/2). Man isolierte 21.5 g (42 %) Produkt mit Schmp. 84-86°C.
f) N-(2-Methyl-1-naphthyl)-piperazin
   13,0 g (82,7 mM) 1-Amino-2-methyl-naphthalin in 100 ml Chlorbenzol wurden mit 14,7 g (82,7 mM) Bis-(2-chlorethyl)-amin x HCl versetzt und 90 h unter Stickstoff bei Rückfluß gekocht. Anschließend engte man den Ansatz ein, verteilte zwischen Methylenchlorid und Wasser bei pH=9 und engte die organische Phase nach Trocknen ein. Das Rohprodukt reinigte man durch Säulenchromatographie (Kieselgel, Laufmittel /THF/Methanol/Ammoniak 85/13/2. Man isolierte 11,6 g (62 %) Produkt.
g) 4-Piperazin-1-yl-isochinolin
   Es wurden 4,51 g (21,7 mM) 4-Bromisochinolin, 4,65 g (25,0 mM) Piperazin-N-carbonsäure-t-butylester, 0,1 g (0,11 mM) Tris-(dibenzylidenaceton)-dipalladium, 0,11 g (0,18 mM) 2,2'-Bis-(diphenylphosphino)-1,1'-binaphthyl und 2,92 g (30,4 mM) Natrium-t-butylat in 50 ml Toluol zusammengegeben und 2 h bei 75°C gerührt. Man gab die Reaktionsmischung auf Eis/Kochsalz, extrahierte mit Essigester, trocknete die organische Phase über Natriumsulfat und entfernte das Lösungsmittel am Rotationsverdampfer. Das Produkt kristallisierte aus, wurde abgesaugt und mit Pentan gewaschen. Man erhielt 5,5 g (81 %) des Boc-geschützten Piperazins (Schmp.: 111°C). Es wurden 5,2 g (16,6 mM) dieser Substanz in 17 ml Dichlormethan aufgenommen und bei 0°C langsam mit 17 ml Dichlormethan aufgenommen und bei 0°C langsam mit 17 ml (0,22 mM) Trifluoressigsäure versetzt. Man ließ 4 h bei 0°C rühren, goß auf Eiswasser und extrahierte mit Dichlormethan. Die wässrige Phase wurde filtriert, alkalisch eingestellt und mit Dichlormethan extrahiert. Nach dem Trocknen über Natriumsulfat und dem weitgehenden Entfernen des Lösungsmittels verdünnte man mit Diethylether und fällte das Hydrochlorid mit etherischer Salzsäure. Man erhielt 3,2 g (67 %) des Produktes mit Schmp. 293 - 294°C.
   Analog e), f) und g) stellte man weitere Piperazinderivate (siehe Beispiele) her, soweit sie nicht literaturbekannt waren (vgl. auch Patentanmeldung DE 19636769.7).

### B Herstellung der Endprodukte

### Beispiel 1

### 3,4-Dihydro-5-methyl-6-dimethylcarbamoyl-3-[2-(4-(2-methoxyphenyl]-piperazin-1-yl)-ethyl]-thieno-(2,3-d]-pyrimidin-4-on

2,4 g (7,8 mM) 2-Ethoxymethylen-amino-3-carboethoxy-4-methyl-5-dimethyl-carbamoyl-thiophen in 30 ml Ethanol wurden mit 1,9 g (8,0 mM) 1-(2-Amino-ethyl)-4-(2-methoxy-phenyl)-piperazin versetzt und 2 h am Rückfluß gekocht. Nach Stehen über Nacht kristallisierte das Produkt aus, das abgesaugt und mit wenig Ethanol gewaschen wurde. Man isolierte 2,2 g (62 %) Produkt mit
Schmp. 188 - 190°C.

### Beispiel 2

### 3,4-Dihydro-5-methyl-6-dimethylcarbamoyl-3-[2-(4-(2,3-dimethylphenyl)-piperazin-1-yl)-ethyl]-thieno[2,3-d]pyrimidin-4-on

1,5 g (5,0 mM) 3-(2-Chlor-ethyl)-5-methyl-6-dimethylcarbamoylthieno[2,3-d]pyrimidin-4-on in 15 ml Dimethylformamid wurden mit 1,1 g (5,0 mM) 1-(2,3-Dimethylphenyl)-piperazin-hydrochlorid sowie 1,54 ml (11 mM) Triethylamin versetzt und insgesamt 3 h auf 125°C unter Stickstoff erhitzt. Nach dem Eingießen in Wasser wurde mit Essigester extrahiert, die organische Phase mit verdünnter Salzsäure bei pH=2 extrahiert und die daraus resultierende wäßrige Phase mit verdünnter Natronlauge basisch gestellt. Man extrahierte das Rohprodukt mit Dichlormethan, trocknete über Natriumsulfat und entfernte das Lösungsmittel im Vakuum. Der ölige Rückstand wurde aus wenig Methanol kristallisiert und abgesaugt. So konnten 0,7 g (31 %) Produkt mit Schmp. 160-161°C erhalten werden.

Analog der Beispiele 1 und 2 wurden hergestellt:
3. 3,4-Dihydro-5-methyl-6-dimethylcarbamoyl-3-[2-(4-(1-naphthyl)-piperazin-1-yl)-ethyl]-thieno[2,3-d]pyrimidin-4-on, Schmp. 190-191°C
4. 3,4-Dihydro-5-methyl-6-dimethylcarbamoyl-3-[2-(4-(2-methyl-1-naphthyl)-piperazin-1-yl)-ethyl]-thieno[2,3-d]pyrimidin-4-on, Schmp. 178-180°C
5. 3,4-Dihydro-5-methyl-6-dimethylcarbamoyl-3-[2-(4-(2-methoxy-1-naphthyl)-piperazin-1-yl)-ethyl]-thieno[2,3-d]pyrimidin-4-on x H₂O, Schmp. 153-155°C (Zers.)
6. 3,4-Dihydro-5-methyl-6-dimethylcarbamoyl-3-[2-(4-(2-methyl-phenyl)-piperazin-l-yl)-ethyl]-thieno[2,3-d]pyrimidin-4-on
7. 3,4-Dihydro-5-methyl-6-dimethylcarbamoyl-3-[2-(4-(3-trifluormethyl-phenyl)-piperazin-1-yl)-ethyl]-thieno-[2,3-d]pyrimidin-4-on, Schmp. 146°C
8. 3,4-Dihydro-5-methyl-6-dimethylcarbamoyl-3-[2-(4-(2-chlor-phenyl)-piperazin-1-yl)-ethyl]-thieno-[2,3-d]pyrimidin-4-on
9. 3,4-Dihydro-5-methyl-6-dimethylcarbamoyl-3-[2-(4-pyrimidin-2-yl-piperazin-l-yl)-ethyl]-thieno-[2,3-d]pyrimidin-4-on x 2HCl x 4 H₂O, Schmp. 180-182°C (Zers.)
10. 3,4-Dihydro-5-methyl-6-dimethylcarbamoyl-3-[2-(4-pyridin-2-yl-piperazin-l-yl)-ethyl]-thieno-[2,3-d]pyrimidin-4-on
11. 3,4-Dihydro-5-methyl-6-dimethylcarbamoyl-3-[2-(4-chinolin-2-yl-piperazin-1-yl)-ethyl]-thieno-[2,3-d]pyrimidin-4-on
12. 3,4-Dihydro-5-methyl-6-dimethylcarbamoyl-3-[2-(4-(3,5-dichlor-phenyl)-piperazin-1-yl)-ethyl]-thieno-[2,3-d]pyrimidin-4-on
13. 3,4-Dihydro-5-methyl-6-dimethylcarbamoyl-3-[2-(4-tetralin-5-yl-piperazin-1-yl)-ethyl]-thieno-[2,3-d]pyrimidin-4-on, Schmp. 174°C
14. 3,4-Dihydro-5-methyl-6-dimethylcarbamoyl-3- [2-(4-indan-4-yl-piperazin-1-yl]-ethyl]-thieno-[2,3-d]pyrimidin-4-on, Schmp. 153°C
15. 3,4-Dihydro-5-methyl-6-dimethylcarbamoyl-3-[2-(4-(2-cyano-phenyl)-piperazin-1-yl)-ethyl]-thieno-[2,3-d]pyrimidin-4-on, Schmp. 210°C (Hydrochlorid)
16. 3,4-Dihydro-5-methyl-6-dimethylcarbamoyl-3-[2-(4-isochinolin-4-yl-piperazin-1-yl)-ethyl]-thieno-[2,3-d]pyrimidin-4-on
17. 3,4-Dihydro-5-methyl-6-dimethylcarbamoyl-3-[3-(4-pyrimidin-2-yl-piperazin-1-yl)-propyl]-thieno-[2,3-d]pyrimidin-4-on x 2 HCl x 2 H₂O, Schmp. 209-211°C (Zers.)
18. 3,4-Dihydro-5-methyl-6-dimethylcarbamoyl-3-[2-(4-(2-methoxy-phenyl)-piperidin-1-yl)-ethyl]-thieno-[2,3-d]pyrimidin-4-on
19. 3,4-Dihydro-5-methyl-6-dimethylcarbamoyl-3-[2-(4-(2-methoxy-phenyl)-3,4-dihydropiperidin-1-yl)-ethyl]-thieno[2,3-d]pyrimidin-4-on
20. 3,4-Dihydro-5-methyl-6-dimethylcarbamoyl-3-[2-(4-naphth-1-yl-piperidin-1-yl)-ethyl]-thieno-[2,3-d]pyrimidin-4-on
21. 3,4-Dihydro-5-methyl-6-dimethylcarbamoyl-3- [2- (4-(2-methoxy-naphth-1-yl)-3,4-dehydropiperidin-1-yl)-ethyl]-thieno[2,3-d]pyrimidin-4-on
22. 3,4-Dihydro-5-methyl-6-dimethylcarbamoyl-3-[2-(4-naphth-1-yl-1,4-hexahydro-1,4-diazepin-1-yl)-ethyl]-thieno[2,3-d]pyrimidin-4-on, Schmp. 225 - 230°C (Hydrochlorid)
23. 3,4-Dihydro-5-methyl-6-carbamoyl-3-[2-(4-(1-naphthyl)-piperazin-1-yl)-ethyl]-thieno[2,3-d]pyrimidin-4-on
24. 3,4-Dihydro-5-methyl-6-carbamoyl-3-[2-(4-pyrimidin-2-ylpiperazin-1-yl)-ethyl]-thieno[2,3-d]pyrimidin-4-on
25. 3,4-Dihydro-5-methyl-6-diethylcarbamoyl-3-[2-(4-(2-methoxyphenyl)-piperazin-1-yl)-ethyl]-thieno-[2,3-d]-pyrimidin-4-on
26. 3,4-Dihydro-5-methyl-6-diethylcarbamoyl-3-[2-(4-(1-naphthyl)-piperazin-1-yl)-ethyl]-thieno[2,3-d]pyrimidin-4-on
27. 3,4-Dihydro-5-methyl-6-diethylcarbamoyl-3-[2-(4-pyrimidin-2-yl-piperazin-1-yl)-ethyl]-thieno[2,3-d]pyrimidin-4-on
28. 3,4-Dihydro-5-methyl-6-dimethylcarbamoyl-3-[2-(4-chinazolin-4-yl-piperazin-1-yl)-ethyl]-thieno-[2,3-d]pyrimidin-4-on, Schmp. 295 - 300°C (Hydrochlorid)
29. 3,4-Dihydro-5-methyl-6-dimethylcarbamoyl-3-[2-(4-(2,4-dimethoxy-phenyl)-piperazin-1-yl)-ethyl]-thieno-[2,3-d]pyrimidin-4-on, Schmp. 170-171°C
30. 3,4-Dihydro-5-methyl-6-dimethylcarbamoyl-3-[2-(4-(2,5-dimethyl-phenyl)-piperazin-1-yl)-ethyl]-thieno-[2,3-d]pyrimidin-4-on, Schmp. 90-91°C
31. 3,4-Dihydro-5-methyl-6-dimethylcarbamoyl-3-[2-(4-naphth-1-yl-3,4-dehydropiperidin-1-yl)-ethyl]-thieno[2,3-d]pyrimidin-4-on, MS: m⁺ = 509,1

## Patentansprüche

1. 3-substituierte 3,4-Dihydro-thieno[2,3d]pyrimidin-Derivate der Formel I 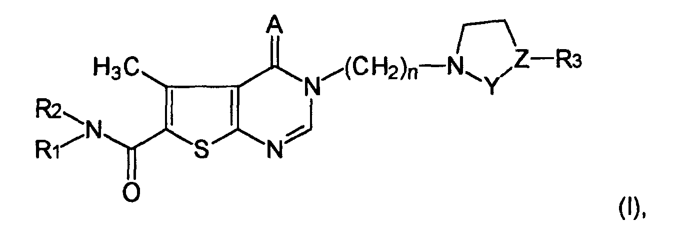 worin
R¹ und R² ein Wasserstoffatom oder eine C₁-C₄-Alkylgruppe bedeutet,
R³ gegebenenfalls durch Halogenatome, C₁-C₄-Alkyl, Trifluormethyl-, Trifluormethoxy-, Hydroxy-, C₁-C₄-Alkoxy-, Amino-, Monomethylamino-, Dimethylamino-, Cyano- oder Nitrogruppen mono-oder disubstituierte Phenyl-, Pyridyl-, Pyrimidinyl- oder Pyrazinyl-Gruppe darstellt, die gegebenenfalls mit einem Benzolkem, der gegebenenfalls durch Halogenatome, C₁-C₄-Alkyl-, Hydroxy-, Trifluormethyl-, C₁-C₄-Alkoxy-, Amino-, Cyano- oder Nitrogruppen mono- oder disubstituiert sein kann und gegebenenfalls 1 Stickstoffatom enthalten kann, oder mit einem 5-oder 6-gliedrigen Ring, der 1-2 Sauerstoffatome enthalten kann, anelliert sein kann,
A NH oder ein Sauerstoffatom darstellt,
Y CH₂, CH₂-CH₂, CH₂-CH₂-CH₂ oder CH₂-CH ist,
Z ein Stickstoffatom, Kohlenstoffatom oder CH darstellt, wobei die Bindung zwischen Y und Z auch eine Doppelbindung sein kann, und
n die Zahl 2, 3 oder 4 bedeutet,
sowie deren physiologisch verträgliche Salze.

2. Verbindungen nach Anspruch 1, **dadurch gekennzeichnet, dass**
R¹ und R² Methyl,
R³ o-Methoxyphenyl, 1-Naphthyl, 2-Methoxy-1-naphthyl, 2-Methyl-1-naphthyl,
A ein Sauerstoffatom,
Y CH₂-CH₂,
Z ein Stickstoffatom und
n die Zahl 2 und 3 bedeuten.

3. Verbindungen nach Anspruch 1 oder 2 zur Verwendung als Arzneimittel.

4. Verbindungen nach Anspruch 1 oder 2 als selektive 5HT_{1B}- und 5HT_{1A}-Antagonisten.

5. Verwendung einer Verbindung nach einem der Ansprüche 1 oder 2 zur Herstellung eines Arzneimittels zur Behandlung von Depressionen, zentralnervös bedingten Gemütsstörungen, Angstzuständen, Gedächtnisstörungen, psychogenen Eßstörungen, endokrinen Erkrankungen, Gefäßspasmen, Hypertonie, gastrointestinalen Störungen und Sexualstörungen.

## Claims

1. 3-Substituted 3,4-dihydrothieno[2,3d]pyrimidine derivatives of formula I: 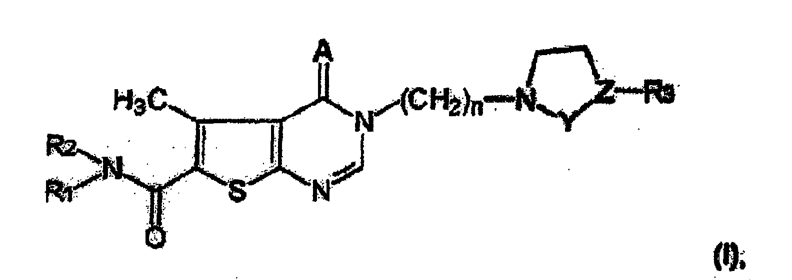 wherein
R¹ and R² are a hydrogen atom or a C₁-C₄-alkyl group,
R³ is a phenyl, pyridyl, pyrimidinyl or pyrazinyl group which is optionally mono- or disubstituted by halogen atoms or C₁-C₄-alkyl, trifluoromethyl, trifluoromethoxy, hydroxyl, C₁-C₄-alkoxy, amino, monomethylamino, dimethylamino, cyano or nitro groups, and which can optionally be fused to a benzene ring, it optionally being possible for the latter to be mono- or disubstituted by halogen atoms or C₁-C₄-alkyl, hydroxyl, trifluoromethyl, C₁-C₄-alkoxy, amino, cyano or nitro groups and to contain 1 nitrogen atom, or fused to a 5- or 6-membered ring that can contain 1-2 oxygen atoms,
A is NH or an oxygen atom,
Y is CH₂, CH₂-CH₂, CH₂-CH₂-CH₂ or CH₂-CH,
Z is a nitrogen atom, a carbon atom or CH, it also being possible for the bond between Y and Z to be a double bond, and
n is the number 2, 3 or 4,
and their physiologically acceptable salts.

2. Compounds according to Claim 1, **characterized in that**
R¹ and R² are methyl,
R³ is o-methoxyphenyl, 1-naphthyl, 2-methoxy-1-naphthyl or 2-methyl-1-naphthyl,
A is an oxygen atom,
Y is CH₂-CH₂,
Z is a nitrogen atom and
n is the number 2 or 3.

3. Compounds according to Claim 1 or 2 for use as drugs.

4. Compounds according to Claim 1 or 2 as selective 5HT_{1B} and 5HT_{1A} antagonists.

5. Use of a compound according to Claim 1 or 2 for the preparation of a drug for the treatment of depression, mood disorders caused by the central nervous system, anxiety states, memory disorders, psychogenic eating disorders, endocrine diseases, vasospasms, hypertonia, gastrointestinal disorders and sexual disorders.

## Revendications

1. Dérivés 3,4-dihydro-thiéno[2,3d]pyrimidine 3-substitués de la formule I 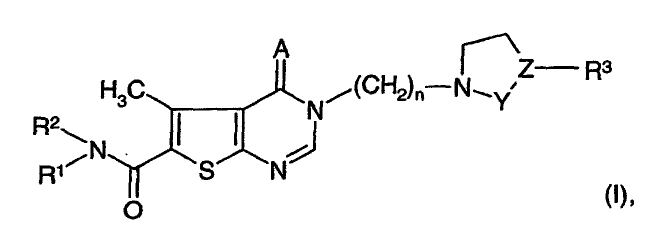 dans laquelle
R¹ et R² représentent un atome d'hydrogène ou un groupe alkyle en C₁-C₄,
R³ représente un groupe phényle, pyridyle, pyrimidinyle ou pyrazinyle éventuellement mono- ou di-substitué par des atomes d'halogène, des groupes alkyle en C₁-C₄, trifluorométhyle, trifluorométhoxy, hydroxy, alcoxy en C₁-C₄, amino, monométhylamino, diméthylamino, cyano ou nitro, lequel peut être éventuellement condensé avec un noyau benzène, qui peut être éventuellement mono- ou di-substitué par des atomes d'halogène, des groupes alkyle en C₁-C₄, hydroxy, trifluorométhyle, alcoxy en C₁-C₄, amino, cyano ou nitro et qui peut éventuellement contenir un atome d'azote, ou avec un cycle à de 5 à 6 éléments qui peut contenir 1-2 atomes d'oxygène,
A représente NH ou un atome d'oxygène,
Y est CH₂, CH₂-CH₂, CH₂-CH₂-CH₂ ou CH₂-CH,
Z représente un atome d'azote, un atome de carbone ou CH, la liaison entre Y et Z pouvant également être une double liaison, et
n représente le nombre 2, 3 ou 4,
ainsi que leurs sels physiologiquement compatibles.

2. Composés selon la revendication 1, **caractérisés en ce que**
R¹ et R² représentent le groupe méthyle,
R³ représente le groupe o-méthoxyphényle, 1-naphtyle, 2-méthoxy-1-naphtyle, 2-méthyl-1-naphtyle,
A représente un atome d'oxygène,
Y représente CH₂-CH₂,
Z représente un atome d'azote et
n représente le nombre 2 et 3.

3. Composés selon la revendication 1 ou 2 destinés à une utilisation comme médicament.

4. Composés selon la revendication 1 ou 2 comme antagonistes 5HT_{1B} et 5HT_{1A} sélectifs.

5. Utilisation d'un composé selon l'une quelconque des revendications 1 ou 2 pour la préparation d'un médicament destiné au traitement de dépressions, de troubles psychiques causés par le système nerveux central, d'états d'angoisse, de dysfonctionnements de la mémoire, de troubles alimentaires psychogènes, de maladies endocrinologiques, de spasmes vasculaires, d'hypertonie, de troubles gastro-Intestinaux et de troubles sexuels.
